# EUROPEAN PATENT APPLICATION

(11) **EP 2 596 782 A2**
(43) Date of publication of application: **29.05.2013**
(21) Application number: 11763960.9
(22) Date of filing: 06.07.2011
(51) Int. Cl.: A61K 8/42, A61K 8/43, A61K 8/49, A61Q 11/00

(54) **COMPOUND FOR TREATING THE BUCCAL CAVITY COMPRISING A CHLORHEXIDINE SALT, A QUATERNARY AMMONIUM SALT AND A COMPLEX ALLANTOIN PANTHENOL SALT**

(30) Priority: 22.07.2010 ES 201031133
(71) Applicant: Laboratorios Kin, S.A., 08018 Barcelona (ES)
(72) Inventor: BALASCH RISUEÑO, Joaquín, E-08018 Barcelona (ES); SANCHO RIERA, Enriqueta, E-08029 Barcelona (ES)
(74) Representative: Curell Suñol S.L.P.
(86) International application number: PCT/ES2011/070491
(87) International publication number: WO 2012/010730

(57) **Abstract**

Compound for treating the buccal cavity comprising a chlorhexidine salt, a quaternary ammonium salt and a complex allantoin and panthenol salt. Compound for treating the buccal cavity, which comprises the following constituents:
[a] a chlorhexidine salt, such as for example chlorhexidine digluconate
[b] a quaternary ammonium salt, such as for example cetylpyridine chloride, and
[c] a complex allantoin and panthenol salt.

It is particularly suitable for preparing toothpastes, mouthwashes, etc.

## Description

### Field of the invention

The invention relates to a compound for treating the buccal cavity.

### State of the art

Compounds for treating the buccal cavity are known that comprise some type of antiseptic, such as for example chlorhexidine digluconate.

Various compounds have been developed that try to offer greater antiseptic efficiency than compounds containing only chlorhexidine. However, there is still the need to develop new compounds having a high antiseptic efficiency and which do not depend only on the chlorhexidine content.

### Summary of the invention

The aim of the invention is to offer a new compound with high antiseptic efficiency. This aim is achieved by means of an antiseptic compound for treating the buccal cavity, characterized in that it comprises the following constituents:
[a] a chlorhexidine salt
[b] a quaternary ammonium salt
[c] a complex allantoin panthenol salt

In fact, it has been observed that the combination of these three active ingredients makes it possible to obtain a compound with particularly high antiseptic efficiency and which, in addition has anti-inflammatory, epithelising, superficial wound healing, and calming properties.

The complex allantoin panthenol salt, with the developed formula: 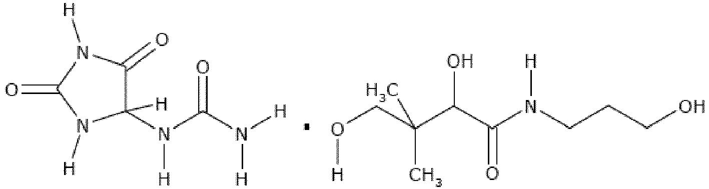 has number CAS 71673-21-7 and it is a molecular complex of allantoin and DL-panthenol. It is obtained by forming a complex salt of both components, in the form of a white powder.

Preferably the quaternary ammonium salt is cetylpyridine chloride. Advantageously the compound comprises between 0.02% and 0.08% cetylpyridine chloride, preferably between 0.04% and 0.06% cetylpyridine chloride.

Preferably the chlorhexidine salt is chlorhexidine digluconate. Advantageously the compound comprises between 0.05% and 0.30% chlorhexidine digluconate, preferably between 0.10 and 0.14% chlorhexidine digluconate. It must be understood that, when it is specified in the description and the claims that the compound has a certain % of chlorhexidine digluconate, it also includes the case wherein the chlorhexidine digluconate has been replaced with the corresponding amount of another chlorhexidine salt.

Preferably the compound comprises between 0.5% and 2% of the complex allantoin and panthenol salt.

A preferred embodiment of the invention is when the compound is a toothpaste and comprises between 1.3 % and 1.7 % of the complex allantoin and panthenol salt.

Another preferred embodiment of the invention is when the compound is a mouthwash and comprises between 0.65 % and 0.85 % of the complex allantoin and panthenol salt.

All the % indicated in this description and claims are by weight and with respect to the compound total.

The aim of the invention is also a packaging, preferably a rinse for the buccal cavity, comprising a body suitable for housing a pharmaceutical or cosmetic compound, an outlet hole and a reversibly removable cap, characterized in that it comprises a compound according to the invention.

The aim of the invention is also a tube of toothpaste comprising a deformable body, an outlet hole and a reversibly removable cap, characterized in that it comprises a compound according to the invention.

### Brief description of the drawings

Other advantages and characteristics of the invention will be appreciated from the following description, wherein, in a non-limiting manner, some preferable embodiments of the invention are described, with reference to the accompanying drawing, wherein:

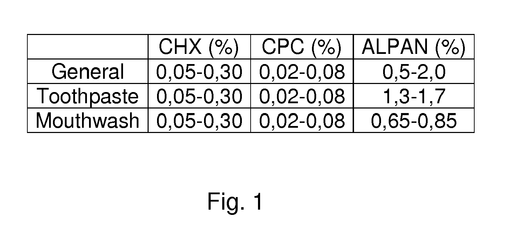

Fig. 1, a table containing some compounds according to the invention.

### Detailed description of some embodiments of the invention

Fig. 1 shows a table with the preferable ranges of chlorhexidine digluconate (CHX column), cetylpyridine chloride (CPC column) and a complex allantoin and panthenol salt (ALPAN column): in the general case (row 1), in the case of a toothpaste (row 2) and in the case of a rinse or mouthwash (row 3).

### Example 1:

### Mouthwash

| **Description** | **100** | **g** |
|---|---|---|
| Chlorhexidine Digluconate 20% | 0,6166 | g |
| Cetylpyridine Chloride | 0,0482 | g |
| Alpantha | 0,7225 | g |
| Non-crystallisable Sorbitol 70 % | 9,6339 | g |
| Glycerol | 4,8170 | g |
| Purified water qs | | |
| | 100,0000 | g |

The amount of sorbitol can vary, and preferably it has values between 5% and 15%. Also, the amount of glycerol can vary, with its preferable values being between 2% and 15%.

In addition, other constituents can be added to the formula, such as sodium saccharin, crystallized menthol, eugenol, methyl salicylate, crystallised citric acid, aromas, preservatives, solubilising agents, etc.

### Example 2:

### Toothpaste

| **Description** | **100** | **g** |
|---|---|---|
| Chlorhexidine Digluconate | 0,120 | g |
| Cetylpyridine Chloride | 0,050 | g |
| Alpantha | 1,500 | g |
| Xylitol | 1,000 | g |
| Non-crystallisable Sorbitol 70 % | 35,000 | g |
| Glycerol | 15,000 | g |
| Excipient qs | | |
| | 100,000 | g |

The amount of sorbitol can vary, and preferably it has values between 10% and 40%. Also, the amount of glycerol can vary, with its preferable values being between 5% and 20%.

In addition, the formula comprises other conventional constituents, such as hydroxyethylcelulose, hydrated silica, sodium saccharin, crystallized menthol, eugenol, methyl salicylate, titanium dioxide, cocamidopropyl betaine, etc.

To carry out both formulae the complex allantoin and panthenol salt has been used, supplied by Akema Fine Chemicals S.r.l. (Via Puglie 12, Coriano (RN) 47853 Italy) under the name of alpantha.

## Claims

1. Compound for treating the buccal cavity, **characterized in that** it comprises the following constituents:
[a] a chlorhexidine salt
[b] a quaternary ammonium salt
[c] a complex allantoin panthenol salt

2. Compound according to claim 1, **characterized in that** said quaternary ammonium salt is cetylpyridine chloride.

3. Compound according to claim 2, **characterized in that** it comprises between 0.02% and 0.08% of cetylpyridine chloride, preferably between 0.04% and 0.06% of cetylpyridine chloride.

4. Compound according to any of the claims 1 to 3, **characterized in that** said chlorhexidine salt is chlorhexidine digluconate.

5. Compound according to claim 4, **characterized in that** it comprises between 0.05% and 0.30% of chlorhexidine digluconate, preferably between 0.10 and 0.14% of chlorhexidine digluconate.

6. Compound according to any of the claims 1 to 5, **characterized in that** it comprises between 0.5% and 2% of the complex allantoin and panthenol salt.

7. Compound according to any of the claims 1 to 6, **characterized in that** it is a toothpaste and comprises between 1.3 % and 1.7 % of the complex allantoin and panthenol salt.

8. Compound according to any of the claims 1 to 6, **characterized in that** it is a mouthwash and comprises between 0.65 % and 0.85 % of the complex allantoin and panthenol salt.

9. Packaging comprising a body suitable for housing a pharmaceutical or cosmetic compound, an outlet hole and a reversibly removable cap, **characterized in that** it comprises a compound according to any of the claims 1 to 8.

10. Tube of toothpaste comprising a deformable body, an outlet hole and a reversibly removable cap, **characterized in that** it houses in the inside thereof a compound according to any of the claims 1 to 8.

11. Packaging for a mouthwash for the buccal cavity comprising a body suitable for housing a pharmaceutical or cosmetic compound, an outlet hole and a reversibly removable cap, **characterized in that** it comprises a compound according to any of the claims 1 to 8.
